# EUROPEAN PATENT SPECIFICATION

(11) **EP 1 903 337 B1**
(45) Date of publication and mention of the grant of the patent: **22.07.2015**
(21) Application number: 06019661.5
(22) Date of filing: 20.09.2006
(51) Int. Cl.: G01N 33/543, G01N 33/58

(54) **Coating for microcarriers**
Beschichtung für Mikroträger
Revêtement pour microporteurs

(43) Date of publication of application: 26.03.2008
(73) Proprietor: Mycartis N.V., 9052 Zwijnaarde / Ghent (BE)
(72) Inventor: De Geest, Bruno, 9051 St. Denjis Westrem (BE); Demeester, Joseph, 9040 Sint-Amandsberg (BE); Derveaux, Stefaan, 9200 Dendermonde (BE); De Smedt, Stefaan, 9030 Mariakerke (BE)
(74) Representative: Jolly, Christophe

(56) References cited:
- WO-A-02/33419
- WO-A-91/09141
- WO-A-99/47253
- WO-A-03/062372
- CN-A- 1 524 925
- US-A1- 2005 208 543
- MALSCH R ET AL: "Protamine and heparin-coated ferromagnetic polystyrene-beads for flow cytometry analysis" ANNALS OF HEMATOLOGY, vol. 70, no. SUPPL. 1, 1995, page A6, XP009089155 & 39TH ANNUAL MEETING OF THE SOCIETY FOR THROMBOSIS AND HEMOSTASIS RESEARCH; BERLIN, GERMANY; FEBRUARY 15-18, 1995 ISSN: 0939-5555
- BRAECKMANS KEVIN ET AL: "Encoding microcarriers: present and future technologies" NATURE REVIEWS. DRUG DISCOVERY, NATURE PUBLISHING GROUP, BASINGSTOKE, GB, vol. 1, no. 6, June 2002 (2002-06), pages 447-456, XP002437064 ISSN: 1474-1784

## Description

### FIELD OF THE INVENTION

The invention relates to a coating for microcarriers, whereby the coating allows orientation of the microcarriers within a magnetic field, e.g. for reading and/or writing of a code and allows optimal binding of reagents thereto. Moreover, the coating of the invention does not interfere with the reading of a code on the microcarrier.

### BACKGROUND OF THE INVENTION

Drug discovery and drug screening commonly involve performing assays on very large numbers of compounds or molecules. These assays typically include screening chemical libraries for compounds of interest, screening for particular target molecules in test samples, and testing generally for chemical and biological interactions of interest between molecules. They thus often imply carrying out thousands of individual chemical or biological reactions. In order to avoid spatial limitations of microtiter plates and allow a more efficient use of reagents, methods have been developed to carry out high-throughput assays and reactions on microcarriers as supports. Each microcarrier may contain one particular ligand bound to its surface to act as a reactant, and the reaction of each microcarrier is tracked by the presence on the microcarrier of a "code" that identifies the microcarrier and therefore identifies the particular ligand bound to its surface. Such encoding allows for "random processing" which means that thousands of uniquely coded microcarriers, each having a particular ligand bound to their surface, may all be mixed and subjected to an assay simultaneously. Those microcarriers that show a reaction of interest between the attached ligand and target analyte can be identified based on their code, thereby providing the information on the ligand that produced the reaction of interest.

Different types of encoded microcarriers have emerged over the years (reviewed in Braeckmans et al. (2002) Nat. Rev. Drug Discov. 1(6), 447-456). An important problem with regard to code encryption and identification is the random positioning of microcarriers and the consequent lack of efficiency of the encoding and identification of the microcarriers. This problem is addressed in WO0233419 by the incorporation of a magnetic material in the coating of the microcarrier, which allows the manipulation of the microcarrier in a magnetic field. The metal-coated beads described in WO0233419 however provide a speckled appearance, which can impede the proper reading of encoded microcarriers. Different types of magnetic microcarriers have been described in the prior art, mainly with the aim of immobilising the particles in certain assay steps or to allow swift separation of the particles from the reagents.

WO03/062372 describes bead having a hollow microstructure with an inner layer of nanoparticles bound to a "structure directing agent" (e.g. polyelectrolytes), and an outer layer of nanoparticles, also bound to the structure to the "structure directing agent". The nanoparticles are typically composed of metals, metal non-oxides, metal oxides and organics. CN 1 524 925 discloses a fluorescent magnetic nanoparticle being sequentially coated with polyelectrolyte multiplayers, magnetic nanoparticles, semiconductor fluorescent nanocrystal and polyelectrolyte multilayers. US 2005/208543 discloses beads coded with phosphor particles. These may have a solid core, being a magnetic or paramagnetic particle. Malsch, R. et al, (1995: Annals of hematology, Vol. 70(1); p. A6), disclose the use of superparamagnetic beads to bind covalently herparin or proteamine onto their surface for flow cytometry analysis.

WO9109141 describes beads coated with a mixture of polymer and metal particles. US6,773,812 describes particles cross-linked with magnetic beads. US6,479,146 describes beads that are coated using layer-by-layer technology, which after removal of the central core, consist of a shell of a plurality of alternating layers of polyelectrolyte molecules and nanoparticles. The nanoparticles are optionally magnetised by the use of a layer of magnetic particles such as Fe₃O₄. None of these beads are described to be encoded and these disclosures do not take into account the interference of the coating with the reading of a code written on the surface of the microcarrier. Alternative coatings which combine magnetic properties for orientation in a magnetic field and limited interference with code detection are needed.

### SUMMARY OF THE INVENTION

It is a particular advantage of the present invention to provide coated microcarriers which can be oriented in a magnetic field and are suitable for encoding.

A first aspect of the present invention relates to a microcarrier characterised in that it comprises a core coated with
- at least one layer comprising a polyelectrolyte material; and
- at least one layer comprising magnetic material comprising particles of less than 500 nanometer, the magnetic material having a concentration ranging from 0.1 to 50% by weight of the microcarrier;
wherein each said at least one layer comprising magnetic material is applied on top of one of said at least one layer comprising polyelectrolyte material and wherein the microcarrier is encoded with a written code wherein said code is a spatial modulation created inside the microcarrier or on its outer surface, said spatial modulation being a known arrangement of a finite number of distinct volume elements.According to one embodiment the microcarrier comprises one single layer of magnetic particles.

In particular embodiments of the invention, the core of the microcarrier comprises a bleachable material.

In particular embodiments, the microcarrier comprises between 2 and 10 layers comprising polyelectrolyte material.

According to a particular embodiment, the outer layer of the microcarrier is a layer comprising negatively charged polyelectrolyte material.

According to a particular embodiment, the magnetic material is ferromagnetic material.

According to particular embodiments, the magnetic particles present in the layer of magnetic material on the microcarriers of the invention have a size between 200 and 400 nanometer.

According to particular embodiments, the microcarriers of the invention have a diameter between 1 and 500 µm, e.g. between 10 and 100 µm.

According to the invention, the magnetic particles may have a size of less than 220 nanometer.

According to further particular embodiments, the microcarriers are encoded.

According to one embodiment, the microcarriers are encoded in the central plane of the microcarrier.

Specific embodiments of the microcarriers of the present invention further comprise one or more probes bound to the outer layer of polyelectrolyte material.

A second aspect of the present invention provides a method for manufacturing magnetic microcarrier comprising the steps of providing a microparticle, applying over the microparticle, a layer comprising a polyelectrolyte material and applying on top of the layer of polyelectrolyte material, a layer comprising magnetic material comprising particles of less than 500 nanometer, the magnetic material having a concentration ranging from 0.1 to 50% by weight of the microcarrier. The method further comprising the step of encoding the microcarrier with a code wherein the code is a spatial modulation created inside the microcarrier or on its outer surface.

In particular embodiments, multiple alternating layers of polyelectrolyte material and magnetic material are consecutively applied, by repeating the previous steps. In specific embodiments, the method additionally comprises adding one outer layer of polyelectrolyte material.

Further particular embodiments of the methods of the present invention, comprise the steps of:
a) providing a microparticle,
b) applying one layer comprising a positively charged polyelectrolyte or applying a plurality of layers comprising electrolytes with alternating charges wherein the outer layer has a positive charge,
c) applying one layer of magnetic particles on the particle obtained in step (b),
d) optionally repeating steps b) and c) one or more times, whereby the one layer comprising a positively charged polyelectrolyte is repeatedly applied on the layer of magnetic particles obtained in previous step (c) and,
e) applying one layer comprising a positively charged polyelectrolyte or applying a plurality of layers comprising electrolytes with alternating charges comprising alternating charges wherein the inner layer has a positive charge.

A further aspect of the present invention relates to the use a microcarrier as described above for manipulating a microcarrier a magnetic field.

A further aspect of the present invention relates to the use of layer-by-layer technology to improve the homogenous distribution of metallic material on an encoded microcarrier.

A further aspect of the present invention relates to the use of a microcarrier as described above for the binding of probes to the outer layer of a microcarrier.

The present invention provides microcarriers comprising a core coated with at least one layer of magnetic particles upon at least one layer of polyelectrolytes, whereby the coating makes the microcarriers particularly suited for use in high throughput assays, as the coating ensures that the binding of probes to the surface is optimised and does not interfere with visual detection or reading of a code on the microcarrier.

The present invention thus makes it possible to combine, in a coating of a microcarrier, a) magnetic properties for manipulation of the microcarrier b) optimisation of the coupling efficiency of capture probes to the microcarriers and c) optimal visualisation of encrypted codes. This is achieved by applying alternate layer(s) of polyelectrolyte material and magnetic particles using methods such as layer-by-layer (LbL) technology.

The invention provides microcarriers that can be positioned in a magnetic field to allow appropriate and enhanced read-out of their code.

### BRIEF DESCRIPTION OF THE FIGURES

The following description, which is not intended to limit the invention to specific embodiments described, may be understood in conjunction with the accompanying Figures, incorporated herein by reference, in which:

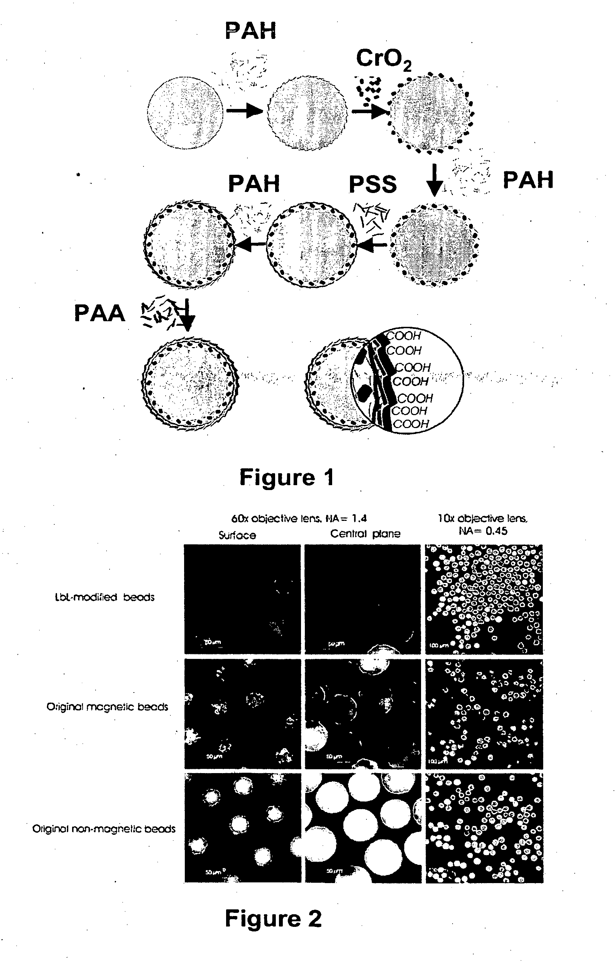

Figure 1 shows a schematic representation of an embodiment of the LbL modification of microcarriers. Charged polymers with opposite charge are sequentially - loaded on the carriers (PAH = poly-allylaminehydrochloride; PSS = poly-styrenesulfonate; PAA = polyacrylic acid). A layer of chromiumdioxide particles (CrO₂, size < 0.45 m) is built in between two layers of positively charged electrolytes.
Figure 2 shows a) the incorporation of chromium dioxide particles (size < 0.45 µm) at the surface of 39 µm-carriers by means of the LbL technology (top row). b) commercial magnetic particles as described in WO0233419 (middle row) and c) uncoated non-magnetic carriers (row 3). Images are taken with a BioRad mrc1064 confocal laser scanning system. Left column: confocal fluorescent image of the top plane (surface plane) of the carriers; Middle column: confocal fluorescent image of the central plane of the carriers; Right column: fluorescent overview image of the carriers.

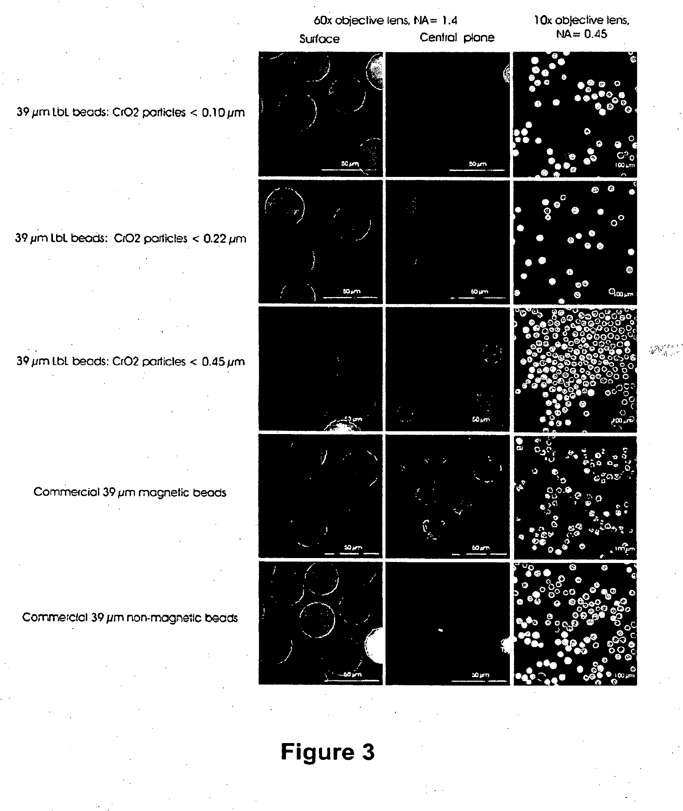

Figure 3 shows examples of the incorporation of chromiumdioxide particles with different size (< 0.1 µm, < 0.22 µm and < 0.45 µm) at the surface of 39 µm microcarriers by means of LbL technology (respectively row 1, 2 and 3). As a comparison, commercial magnetic carriers (row 4) and non-coated microcarriers (row 5) are depicted. Images are taken with a BioRad mrc1064 confocal laser scanning system. Left column: confocal fluorescent image of the central plane of the microcarriers; Middle column: confocal fluorescent image of the top plane (surface plane) of the microcarriers; Right column: fluorescent overview image of the microcarriers. Excitation wavelength = 488 nm.

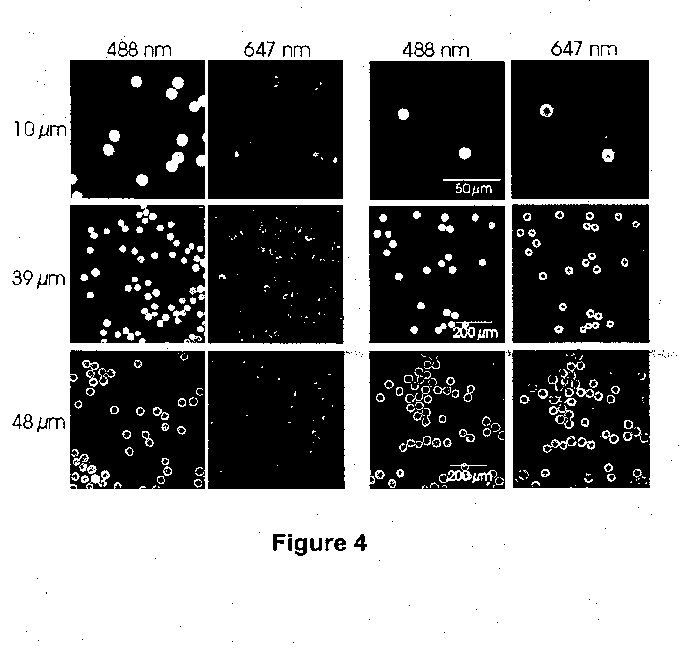

Figure 4 shows the effect of LbL modification of different types of commercially available microcarriers on the binding efficiency of a Cy5 labelled probe (amino-tagged 20-mer oligonucleotides are bound via a carbodiimide to the carboxyl groups of PAA). Left part: non-coated microcarriers; right part: LbL modified microcarriers. First row: 10 µm sized microcarriers; second row: 39 µm sized microcarriers; third row: 48 µm sized microcarriers. Microcarriers are excited with a 488- nm laser wavelength (columns 1 and 4); Cy5-labeled probes are excited with 647- nm wavelength (columns 2 and 3).

### DETAILED DESCRIPTION OF THE INVENTION

In a first aspect, the invention provides a coating for microcarriers which allows positioning and orientation of the microcarriers in a magnetic field without affecting visibility of the core of the microcarrier or a code provided thereon.

As used herein a "microcarrier" also termed "microsphere", "bead" relates to a particle of a size in the range of 1 µm to 500 µm, suitable for carrying one or more probes.

The term "magnetic" as used herein includes all types of material that respond to a magnetic field, such as, but not limited to ferromagnetic, paramagnetic, and supermagnetic materials.

The term "polyelectrolyte" as used herein encompasses both synthetic and natural polyelectrolytes.

In the context of the present invention, when reference is made to 'outer' layer, it is intended to refer to the layer most removed from the core, and which itself is not covered by one of the layers of the coating of the invention.

The present invention relates to coated microcarriers. Typically, below the coating, the microcarriers contain a "core" or "central part" which functions as a reaction volume or a support. This core may be produced from any material that is routinely employed in high-throughput screening technology and diagnostics. For example, the core of microcarriers may be made from a solid, a semi-solid, or a combination of a solid and a semi-solid, and can be supports such as chemical and biological assays and syntheses. Non-limiting examples of these materials include cellulose, carboxymethyl cellulose, hydroxyethyl cellulose, agar, pore-glass, silica gel, polystyrene, brominated polystyrene, polyacrylic acid, polyacrylonitrile, polyamide, polyacrolein, polybutadiene, polycaprolactone, polyester, polyethylene, polyethylene terephthalate, polydimethylsiloxane, polyisoprene, polyurethane, polyvinylacetate, polyvinylchloride, polyvinylpyridine, polyvinylbenzylchloride, polyvinyltoluene, polyvinylidene chloride, polydivinylbenzene, polymethylmethacrylate, polylactide, polyglycolide, poly (lactide-co-glycolide), polyanhydride, polyorthoester,polyphosphazene, polyphosophaze, polysulfone, grafted copolymer such as polyethylene glycol/polystyrene, cross-linked dextrans, methylstyrene, polypropylene, acrylic polymer, carbon, graphite, polycarbonate, polypeptide, hydrogels, liposomes, proteinaceous polymer, titanium dioxide, latex, resin, lipid, ceramic, charcoal, metal, bentonite, kaolinite, rubber, polyacrylamide, latex, silicone, e.g., polydimethyldiphenyl siloxane, dimethylacrylamide, and the like or combinations thereof are acceptable as well. According to a particular embodiment, the core of the microcarrier is itself a microparticle, i.e. a solid or semi-solid particle. Most particularly, the core used for the generation of the microcarrier according to the invention is a microparticle made of latex, polystyrene, or cross-linked dextrans.

The size and shape of the microcarrier are not critical to the present invention. Most particularly, the microcarriers of the present invention are of a shape and size that is suitable for encoding, positioning and orienting thereof. For example, the microcarriers may be in the form of spheres, or, for example, cylindrical or oval. When spherical in shape, the microcarriers typically have a diameter of 1 to 300 µm. Particular embodiments of the present invention relate to microcarriers having a diameter of 1 to 200 µm. Most particularly, the average size of the microcarriers of the present invention ranges from 10 to 100 µm.

According to the present invention, the coating of the microcarriers of the present invention comprises at least one layer of magnetic particles. This allows their manipulation in a magnetic field.
According to a particular embodiment of the present invention, the magnetic nanoparticle is a superparamagnetic or paramagnetic particles, although ferromagnetic metal oxide can also be used.

Typically, the magnetic particles are metal oxide particles, such as but not limited to chromium oxide, or ferric oxide particles. Particular examples of magnetic particles include particles of Cr₂0₃, Fe₂0₃, Fe₃0₄, Ni-and Co-metals, other metal oxides and metals. The magnetic material can represent a ratio ranging from 0.1 to 50 % by weight of the microcarrier, or represent a weight ratio of the microcarrier ranging from 0.5 to 40 %, or for example at a concentration ranging from 1 to 30%.. In particular embodiments the density of the particles in the coating solution is between 0,5 and 20%.

The present invention relates to microcarrier coatings which comprise at least one layer of magnetic particles, which coatings are characterised by the feature that they do not interfere with the reading of a code provided on or in the core of the microcarrier. The visibility of the inner core of the microcarrier is affected by the size of the magnetic particle used in the coating. According to the present invention, the size of the magnetic particles in the coating is less than 500 nm, more particularly less than 400 nm. According to a particular embodiment, the size of the magnetic particles is between 50 and 400 nm, more particularly between 100 and 400 nm. Magnetic particles suitable for use in the coating of the present invention are available commercially (e.g. SIGMA-ALDRICH, FLUKA). Optionally, such stock solutions are ultrasonicated. Magnetic particles can be obtained by producing a metal oxide stock solution with an average size of less than 500 nm. This can be done by heating and precipitating a mixture of divalent and trivalent metal salts in a ratio of divalent versus trivalent metal salt varying from 0.5 to 2.0. The solution of magnetic particles can optionally be filtered through an 0.1 µm, 0.22 µm, or 0.45 µm pore filter.

According to the present invention, a coating is provided which comprises at least one layer of magnetic particles deposited onto a layer of polyelectrolytes.

The polyelectrolytes envisaged in the context of the present invention include both synthetic and natural polyelectrolytes. Synthetic polyelectrolytes envisaged within the context of the present application include, but are not limited to sodium polystyrene sulfonate (PSS), polyallylamine hydrochloride (PAH), polydiallyldimethyl-ammonium chloride (PDDA), polyacrylamide-co-diallyldimethylammonium chloride, polyethyleneimine (PEI), polyacrylic acid (PAA), polyanetholesulfonic acid, polyvinyl sulfate (PVS), and polyvinylsulfonic acid. Such materials, however, are not generally useful for certain biomedical applications because they are potentially antigenic or toxic. Depending on the type of application biocompatible polyelectrolytes can be used such as hyaluronan, chitosan or charged polypeptides.

Polyelectrolytes are polymers whose repeating units bear an electrolyte group. These groups will dissociate in aqueous solutions (water), making the polymers charged.

Polyelectrolytes which bear cationic groups (positively charged, e.g. poly-L-lysine (PLL), poly(ethylenimine) (PEI), poly(dimethyldiallylammonium chloride) (PDDA), poly(allylamine) (PAH), polylysine, chitosan) are referred to as polycations; polyelectrolytes bearing anionic groups (negatively charged, e.g. succinylated PLL (SPLL), poly(styrenesulfonate) (PSS), poly(vinylsulfate), poly(acrylic acid), heparin, DNA) are referred to as polyanions.

According to the present invention, a coating is provided comprising one or more layers of polyelectrolytes and at least one layer of magnetic particles. Optionally, multiple layers of polyelectrolyte form a polyelectrolyte multilayer (PEM), within the coating.

According to a particular embodiment, the coating of the present invention comprising one or more subsequent layers of polyelectrolytes and magnetic particles is provided onto the core of the microcarrier using a layer-by-layer (LbL) deposition technique. During LbL deposition, a suitable growth substrate (usually charged) is dipped back and forth between dilute baths of positively and negatively charged polyelectrolyte solutions. During each dip a small amount of polyelectrolyte is adsorbed and the surface charge is reversed, allowing the gradual and controlled build-up of electrostaticly cross-linked films of polycation-polyanion layers. According to the invention, at least one layer of polyelectrolyte in the LbL coating is substituted by a layer of charged magnetic nanoparticles. The deposition of polyelectrolytes by LbL technology is for example described in the review of Peyratout & Dahne (2004) Angew Chem Int Ed Engl. 43, 3762-3783. LbL techniques wherein metal layers are deposited on microcarriers are described in US6479146. LbL deposition can also be performed using hydrogen bonding instead of electrostatics.

According to a particular embodiment of the coating of the present invention comprises a first layer of polyelectrolyte, most particularly of positively charged polyelectrolyte, such as, but not limited to PAH or PEI, followed by a layer of magnetic particles. According to a further particular embodiment, the coating further comprises, on top of the layer of magnetic particles one or more additional layers (alternating in charge) of polyelectrolyte and/or magnetic particles. Most particularly the first layer of magnetic particles is provided between two layers of positively charged polyelectrolytes and further layers of oppositely charged polyelectrolytes (and optionally magnetic particles) are alternated. According to a particular embodiment of the invention, the total number of layers in the coating comprises between 2 to 10. While one layer of magnetic particles can suffice for the orientation purposes of the present invention, it is also envisaged that several layers of metal particles can be present in the coating of the microcarrier, without significantly affecting the light emitted by the core of the microcarrier to a level which impedes the reading of a code on such a microcarrier. According to a particular embodiment of the invention, the LbL coated microcarrier further comprises, on its external surface, a layer of polymer. However, in general the LbL coated microcarriers of the present invention have a polyelectrolyte layer as outer layer. The choice of the charge of the outer layer can be selected depending on the desired application of the microcarrier. Polyelectrolyte layers allow efficient adherence of one or more probes to the surface of the microcarrier.

According to the present invention, the one or more polyelectrolyte layer(s) present in the provided coatings, allow optimal attachment of a probe to the surface of a microcarrier. The term 'probe' as used herein refers to any biological or chemical molecule of use in a reaction that can take place on the microcarrier. Typical probes include proteins (antibodies, receptors or receptor ligands), DNA (e.g. oligonucleotides), RNA, carbohydrates, small molecules, enzyme inhibitors, enzyme substrates, pharmaceutical compounds from a library etc. According to a particular embodiment of the invention, the probe is attached to a polyelectrolyte layer of the coating. This attachment can be either directly via opposite charges, or via linkers, which react with a functional group on the polylectrolyte layer and a functional group on the target. The probe can be applied to the electrolyte layer in a separate step, after the coating of the electrolyte layer onto the microcarrier. Alternatively, one or more probes can be incorporated into an electrolyte layer of the coating during the LbL coating of the microcarrier. Probes can be added onto or incorporated into one or more inner or outer layers of electrolyte in the coating. According to a particular embodiment, the probe is attached to the outer electrolyte layer of the coating of the invention.

Thus, a further aspect of the invention provides LbL-coated microspheres comprising at least one layer of magnetic particles of less than 500 nm and at least one layer of polyelectrolyte and, optionally, present thereon, one or more probes for use in a biological or chemical reaction.

The LbL layered microcarriers can be used in a wide range of assays, including, but not limited to, assays wherein a target in a sample is detected by a probe which is attached to the microcarrier coating. The reaction between the probe and its target can be a binding between the probe and the target (e.g. avidin/biotin, antibody/antigen, antibody/hapten, receptor/ligand, sugar/lectin, complementary nucleic acid (RNA or DNA, or combination thereof)) but can also be a chemical reaction between the probe and a target or vice versa (e.g. enzyme/substrate, enzyme/cofactor, enzyme/inhibitor) and/or immunoglobulin/Staphylococcal protein A interaction.

According to the present invention a coating is provided for microcarriers which is optimally suited for reading and/or writing a code thereon. In the context of the present invention, the codes envisaged encompass any spatial modulation created inside the microcarrier or on its outer surface. This spatial modulation may be defined as a known arrangement of a finite number of distinct volume elements located inside or on the surface of the microcarrier. The known arrangement of distinct volume elements can be generated by (i) changing one or more properties of the material in an individual volume element, or (ii) by removing material from an individual volume element, or (iii) by depositing material on an individual volume element, or (iv) by leaving an individual volume element unchanged, or a combination of the above possibilities. This known arrangement for example, may be such that these volume elements lie on one or more dimensions such as on a line arrangement or in a plane. Any reference in this application to codes written "on" the microcarriers thus includes codes written on the surface of the microcarriers as well as codes written at an internal depth of the microcarriers.

According to a particular embodiment of the invention, the coated microcarriers are provided with a code at an internal depth of the microcarriers, more particularly in the centre plane of the microcarriers. Depending on the shape of the microcarrier, writing the code on the centre plane can be advantageous as it provides the largest surface area available for writing. Furthermore, for microcarriers having curved surfaces, writing the code on the centre plane may also be advantageous in that the flat plane facilitates reading and or writing compared to the curved surface.

The codes of the present invention may be of any geometry, design, or symbol that can be written and read on the microcarriers. For example, the codes may be written as numbers or letters, or as codes in the form of symbols, pictures, bar codes, ring codes, or three-dimensional codes. Ring codes are similar to bar codes, except that concentric circles are used rather than straight lines. A ring may contain, for example, the same information as one bar.

According to a particular embodiment of the invention, the microcarriers are encoded using the methods described in WO063695. According to this embodiment, the microcarriers contain a bleachable substance, and the codes on the microcarriers are in the form of bleached patterns within the bleachable portions of the microcarriers. The microcarriers may contain the bleachable substance either on the surface of the core of the microcarrier or also within the core of the microcarrier. The bleachable substance can be mixed with the core material upon generation of the core particles of the microcarrier or can be applied to the core of the microcarrier as a separate layer, optionally by specifically linking bleachable molecules to the surface of the core material. Alternatively, also LbL coated particles are envisaged which have an additional polymer coating on the outside, that can contain a bleachable substance. Bleachable substances particularly envisaged within the context of the invention include bleachable fluorescent or electromagnetic radiation absorbing substances. The microcarriers may contain bleachable luminophores. Examples of luminophores that can be used include fluorescers, phosphorescers, or scintillators. Bleachable chemiluminescent, bioluminescent, or colored substances may be used. The bleachable substances may be, more specifically, fluorescein isothiocyanate ("FITC"), phycoerythrines, coumarins, lucifer yellow, and rhodamine. Alternative embodiments of the bleachable substances include 3-Hydroxypyrene 5,8,10-Tri Sulfonic acid, 5-Hydroxy Tryptamine, 5-Hydroxy Tryptamine (5-HT), Acid Fuhsin, Acridine Orange, Acridine Red, Acridine Yellow, Acriflavin, AFA (Acriflavin Feulgen SITSA), Alizarin Complexon, Alizarin Red, Allophycocyanin, ACMA, Aminoactinomycin D, Aminocoumarin, Anthroyl Stearate, Aryl-or Heteroaryl-substituted Polyolefin, Astrazon Brilliant Red 4G, Astrazon Orange R, Astrazon Red 6B, Astrazon Yellow 7 GLL, Atabrine, Auramine, Aurophosphine, Aurophosphine G, BAO 9 (Bisaminophenyloxadiazole), BCECF, Berberine Sulphate, Bisbenzamide, BOBO 1, Blancophor FFG Solution, Blancophor SV, BodipyFI, BOPRO1, Brilliant Sulphoflavin FF, Calcien Blue, Calcium Green, 99.7%. The bleachable substances should be chosen so that, when bleaching occurs, the code remains on the microcarrier for the period of time that is desired for the use of the microcarriers and any necessary reading of the codes. Thus, a certain amount of diffusion of non-bleached molecules into the bleached areas is acceptable as long as the useful life of the code is preserved.

Codes bleached on microcarriers may also be written to have different intensities of fluorescence or colour within bleached areas of the microcarriers. For example, a bleached coding may contain several different degrees of bleaching, thereby having several different intensities of fluorescence within the bleached region as a whole. Thus, microcarriers may be encoded not only by the geometry of the pattern bleached on the microcarriers, but also by the use of different fluorescent intensities within the pattern.

According to the present invention, a code can be written on the microcarriers, for example, by using a high spatial resolution light source, such as a laser, a lamp, or a source that emits X-rays, alpha and beta rays, ion beams, or any form of electromagnetic radiation. Alternatively, the codes can be written on the microcarriers through photochroming or chemical etching. According to a particular embodiment, the microcarriers of the present invention the microcarriers are encoded using a high spatial resolution light source, and in particular a laser or a lamp in combination with a confocal microscope, as described in WO0063695.

According to yet another embodiment of the invention, the microcarriers are encoded by deposition of material onto the surface of the microcarrier, more particularly the core of the microcarrier. Examples of methods of deposition of codes include but are not limited to laser deposition and electrochemical deposition. Examples of material which can be used for such deposition include any organic compound or material; any inorganic compound or material; a particulate layer of material or a composite material; polymeric materials; crystalline or non-crystalline materials; amorphous materials or glasses; carbonaceous material such as, for example, graphite particles or carbon nanotubes; metallic material, such as, for example, gold, silver, copper, nickel, palladium, platinum, cobalt, rhodium, iridium; any metalchalcognide; metal oxide such as for example, cupric oxide, titanium dioxide; metalsulfide, metalselenide, metal telluride, metal alloy, metal nitride, metalphosphide, metalantimonide, semiconductor, semi-metal. Said material can be deposited in the form of particles such as micro- or nanoparticles. For example, the particles are nanoparticles, that is, typically, particles in the size range of 10 nm to 1000 nm.

A further aspect of the invention thus provides coated microcarriers wherein each microcarrier is differentially encoded. The encoded coated microcarriers of the present invention provide the advantage that the magnetic coating allows positioning/and or orientation of the microcarrier, and improved identification of the code on the microcarrier. The coating of the microcarrier according to the present invention does not affect the visibility of the code present thereon. Moreover, according to a particular embodiment of the invention, the coating of the encoded microcarrier is applied using LbL technology, whereby one or more polyelectrolyte layers are deposited onto the microcarrier, resulting in an optimal binding of probes to the surface of the microcarrier.

The invention provides a coating for microcarriers, which allows positioning and/or orientation of the microcarrier in a magnetic field. This is of particular interest for the reading and writing of magnetic codes onto the microcarrier.

Thus, a further aspect of the present invention provides a method for the manipulation of microcarriers wherein an improved reading is achieved after positioning and/or orientation of the microcarriers coated according to the present invention. Thus the invention provides a method for the manipulation for an identification purpose of a magnetically coated microcarrier comprising the following steps (a) an identification purpose step of the microcarrier; and (b) a positioning and/or orientation step, which occurs prior to or during the identification purpose step. According to one embodiment of the invention the positioning and/or orientation step restricts the rotational movement of the microcarrier as a result of a magnetic field imposed on the microcarrier. Alternatively, the positioning and/or orientation step restricts the rotational movement of the microcarrier as a result of an electrical field imposed on the microcarrier.

According to yet a further embodiment of the invention the positioning and/or orientation step comprises the distribution of the population of microcarriers in a one-layer system and restricting the rotational movement of the microcarriers. In another embodiment of the method of invention the positioning and/or orientation step comprises distribution of the population of microcarriers in a plane configuration having two dimensions. In yet another embodiment of the method of the invention the positioning and/or orientation step comprises a distribution resulting in a line configuration. A one-dimensional configuration results in a faster detection.

According to another embodiment of the invention the magnetic field is for the purposes of orientation only and the distribution step is caused by transportation of the microcarriers using other means, such as, but not limited to a laminar flow pattern in a liquid, gaseous or semi-solid environment. Transport of the microcarrier results in the possibility that the detection means can have a fixed position, thereby further improving the detection speed and dismissing any calibration of the detection means. The laminar flow pattern in a liquid environment can be provided in a capillary tube or can be ensured by micro-sized cilia ensuring the movement of fluid and the particles therein. Besides the laminar flow pattern, other flow patterns are also envisaged. Another embodiment of the invention is a method, wherein the distribution step is ensured by the positioning of the microcarriers in a semi-liquid or a liquid support, wherein said semi-liquid or liquid support may have a differential viscosity or density or can be composed of two or more semi-liquid or liquid layer with different viscosity or density. The microcarrier may then float or be positioned on or in the support at the interface of a viscosity or a density change. The position may vary according to the microcarrier density. The absence of a flow in said distribution of the microcarrier results in the possibility that the detection means could be mobile.

According to the present invention, the magnetically coated microcarriers are positioned and/or oriented in reference to the writing instrument and the reading instrument, such that knowledge on the position and orientation of the microcarrier allows the writing instrument to generate the code, which code can subsequently be reliably resolved by the reading instrument using said knowledge on the position and orientation of the microcarrier on which the code is written. The orientation may be done with reference to one, two, or all three axes, depending on the symmetry of the code. If the code is designed to be symmetric around one or more axes, the microcarrier does not need to be oriented with reference to rotation around these axes. Knowledge on the position and/or orientation of the microcarrier is essential to facilitate the writing and/or reading of a code on the microcarrier, in particular when the identification purpose step(s) are performed in a high throughput application.

The invention further relates to methods of manufacturing a microcarrier which method comprises providing a core particle and coating the core particle with a coating comprising a first layer comprising polyelectrolyte and a second layer comprising magnetic particles of a size of less than 500 nm. The method of manufacture further optionally comprises additional steps in which further layers are coated onto the microcarrier, such as a second layer comprising electrolytes. According to a particular embodiment of the invention the first and second layer comprising electrolytes comprise positively charged electrolytes. The method can further optionally comprise additional steps wherein additional layers of electrolytes are added to the microcarrier, most particularly layers of oppositely charged electrolytes are alternated. A further embodiment of the invention comprises one of the methods described above whereby an additional step is provided for the incorporation/attachment of one or more biological probes to the microcarrier. According to yet a further particular embodiment of the invention the method comprises providing a core particle comprising a bleachable material.

A further aspect of the invention relates to a method for encoding of the coated microcarrier of the present invention which method comprises the step of a) positioning and/or orienting the coated microcarrier in a magnetic field and b) applying the code to the microcarrier. According to a particular embodiment of the invention the microcarriers contain a bleachable substance, and encoding is ensured by high spatial resolution light beam resulting in bleached patterns within the bleachable portions of the microcarriers.

Yet a further aspect of the invention relates to a method for providing a coded magnetic microcarrier which method comprises the steps of providing a microcarrier as described above and the steps of encoding the microcarrier as described above.

Yet a further aspect of the invention relates to an assay to determine the interaction between a probe and a target which method comprises the following steps, not necessarily in the provided order: a) providing a coated encoded magnetic microcarrier according to the present invention, which microcarrier comprises the probe; b) contacting the microcarrier with a solution comprising the target; c) positioning and/or orienting the microcarrier in a magnetic field e) assessing whether or not an interaction has occurred between the probe and the target, and e) identifying the microcarrier based on its code. The method of the invention can further comprise additional steps which include, but are not limited to steps which allow the binding of further reagents to the microcarrrier (e.g. for visualisation of the interaction between target and probe), washing steps, selection steps etc...

Accordingly, the present invention provides for different applications of the microcarriers described herein, such as, but not limited to detection and/or quantification methods. Typically, such uses involve the presence of a probe, specific for an analyte to be detected and/or quantified in a sample, on the surface of the microcarriers. Detection methods in which the microcarriers of the present invention can be used include, but are not limited to different immunological assays (based on antibody-antigen interaction), chemical assays (based on enzyme-substrate interaction) and other binding assays (e.g. based on receptor/ligand interaction).

Yet a further aspect of the invention provides the combination of two or more of the reagents required in the manufacturing and/or the encoding of the magnetic microcarriers described herein and/or for their use. The reagents can be provided in the form of a kit with individually packaged components which are either dry or in solution. The kit can further comprise specific instructions for performing the methods according to the present invention. Particularly such a kit can comprise core particles of the microcarrier, coated magnetic microcarriers, encoded magnetic microcarriers and/or encoded magnetic microcarriers comprising one or more probes.

### EXAMPLES

### Example 1: Layer-by-Layer (LbL) coating procedure of microcarriers with ferromagnetic material.

Magnetic particles (chromiumdioxide) with an average length of 200 nm are harvested from commercially available magnetic particle stocks (SIGMA-ALDRICH) by ultrasonication during 15 minutes and subsequent centrifugation or can be directly prepared from a 1% chromium dioxide stock solution (particle size 100 to 400 nm). The solution with magnetic particles is filtered trough a filter with 0.45 µm pores.

LbL coating of microcarriers with 6 layers of polyelectrolytes was performed with the following stock solutions:
- PAH: poly (allylamine hydrochloride); 2 mg/ml in 0.5M NaCl.
- PSS: poly (styrene sulfonate); 70 kDa; 2 mg/ml in 0.5 M NaCl.
- PAA: poly (acrylic acid); 1 mg/ml in 0.5M NaCl

All polymers were purchased from Sigma Aldrich, Steinheim Germany.

A solution of about 300,000 non-magnetic microparticles was centrifuged during 30 seconds at 4000 rpm in a microcentrifuge (Eppendorf type 5415 D). (suspension of polystyrene Sphero^{™} Green Fluorescent Carboxyl carriers with a diameter of 39 µm (excitation = 488 nm) (Spherotech Libertyville, IL).

1 ml of PAH (poly-allylamine hydrochloride) was added to the pelleted microcarriers, and mixed to maintain the microcarriers in suspension. The suspension was further incubated during 15 minutes at 1500 rpm on a vortex (LAYER 1).

The carriers were washed twice with 1 ml 0.05% Tween20 in distilled water. 1 ml of filtered chromium dioxide particles was added to the carriers, and mixed to until the microcarriers were in suspension. The suspension was further incubated during 15 minutes at 1500 rpm on a vortex (LAYER 2).

The microcarriers were washed twice with 1 ml 0.05% Tween20 in distilled water. 1 ml of PAH (poly-allylamine hydrochloride) was added to the microcarriers, and mixed to maintain the carriers in suspension. The suspension was further incubated during 15 minutes at 1500 rpm on a vortex (LAYER 3). The carriers were washed twice with 1 ml 0.05% Tween20 in distilled water.

1 ml of PSS (poly (styrene sulfonate)) was added to the carriers, and mixed to maintain the carriers in suspension. The suspension was further incubated during 15 minutes at 1500 rpm on a vortex (LAYER 4). The carriers were washed twice with 1 ml 0.05% Tween20 in distilled water. 1 ml of PAH ((poly-allylamine hydrochloride)) was added to the carriers, and mixed to maintain the carriers in suspension. The suspension was further incubated during 15 minutes at 1500 rpm on a vortex (LAYER 5).

1 ml of PAA (poly(acrylic acid)) was added to the carriers, and mixed to maintain the carriers in suspension. The suspension was further incubated during 15 minutes at 1500 rpm on a vortex (LAYER 6). The carriers were washed twice with 1 ml 0.05% Tween20 in distilled water. The pellet was resuspended in 1 ml 0.05% Tween20 in distilled water. These particles are stable for at least 6 months.

As an optional additional step the layers can be cross-linked to achieve a prolonged stability of the LbL coating. This can be done for example by incubating the microcarriers in 1 ml of a fresh-made EDC cross-linking solution during 15 minutes at 21 °C at 1500 rmp. This cross-linking solution contains 100 mg/ml EDC in 0.4M MES-buffer pH 5.0. (EDC = 1-ethyl-3-(3-dimethylaminopropyl) carbodiimide HCl ; MW: 191.7 g/mol); MES : 2-[N-morpholino]ethanesulfonic acid (Sigma Aldrich, Steinheim, Germany). Beads with cross-linked LbL are protected from light and stored at 4°C.

### Example 2: Distribution and concentration of metal particles.

The distribution of magnetic particles as obtained via the LbL technology of the present invention, is significantly more homogeneous than with conventional methods of metal incorporation into polymeric carriers (see Figure 2, column 2, rows 1 and 2).
The shading by metal speckles in the commercially available carriers impedes the correct readout of a code, which has been photobleached in the central plane. No shadows of magnetic particles are observed when the magnetic particles are applied as a layer using LbL technology. This facilitates the correct decoding of a code.

In addition, the loading of metal particles according to the present invention appears to be equal for each carrier, since all the carriers show about the same fluorescence intensity in figure 2 (middle row). This is not the case for the prior art carriers as can be seen in figure 2 (row 2, column 3) where a huge variations in intensity exist between different carriers, due to the different amounts of magnetic particles being present.

### Example 3: Effect of metal particle size on carrier fluorescence.

Different sizes of chromium dioxide particles (particles with a size < 0.10 µm, <0.22 µm and < 0.45 µm) were loaded on 39 µm microcarriers using the methodology as described in example 1. Figure 3 shows that the use of particles with a different size of all result in a homogenous coating. Furthermore, in each case the carriers could be positioned after magnetisation. When particles are coated with metal particles with a diameter lower than 0.22 µm, there is hardly any difference in fluorescence compared to carriers without metal coating.

### Example 4: Effect of LDL laver coating on the binding capacity of biomolecules.

Modification of the microcarriers with the LbL technology has a significant effect on the capture efficiency of biomolecules to their surface. Different types of commercially available carriers were compared with and without LbL modification.

In this experiment, the binding efficiency of a Cy5 labelled probe, an amino-tagged 20-mer oligonucleotide which binds via a carbodiimide to the carboxyl groups of PAA, was investigated. 1 pmol of a Cy5 labelled amino-modified 20-mer oligo was bound to the surface of about 1,000 10 µm, 39 µm and 44 µm sized microcarriers, either as such or after LbL coating. It was found that the intensity of the captured probe at the surface is about 40 to 50 times higher with LbL modified carriers compared to non-LbL modified magnetic carriers (see Figure 4).

After coupling 10 fmol of the same probe to the surface of 1000 LbL modified carriers, the intensity was even still 1.5 times higher than that of the non-LbL coated carriers, which were coupled with 1 pmol (100 times more) of the same probe.

### Example 5: Orientation and positioning of ferromagnetic microcarriers.

The microcarriers of the present invention are magnetised and oriented in an external magnetic field. In these experiments a pattern has been bleached at the central plane of a magnetised magnetic microcarrier while said microcarrier is exposed to and oriented by an external magnetic field. Then the pattern is imaged while the carrier is exposed to a moving external magnetic field. It is tested whether the original orientation-known from the bleached pattern-could be found again after random movement of the microcarrier when said microcarrier was subjected again to the original magnetic field.

A reservoir is made by gluing a plastic cylinder of 0.5 cm diameter onto a microscope cover glass. The reservoir is filled with 80 µl of the microcarrier suspension as prepared in example 1 and the microcarriers were allowed to sediment on the cover glass. The reservoir was then placed above a strong permanent magnet for 1 minute to allow the microcarriers to be magnetised. Next the reservoir is placed on a Bio-RadMRC1024 confocal microscope which is attached to an inverted microscope so that it is possible to use a Nikon 60x water immersion lens to look at the carriers through the bottom cover glass.

A strong permanent magnet is placed at a 20 cm distance from the reservoir in order to orient the carriers without changing their magnetic polarisation. An arrow is bleached at the central plane of a magnetic microcarrier oriented by the external magnetic field from the first strong magnet, thus indicating its original orientation. Next, the confocal microscope is set to take a series of 50 images with a 1,2 second interval between each image. While taking this series of images, a second magnet is used to move around the reservoir: first 90 to one side, then 180 in the opposite direction with the first magnet still in place. Finally the second magnet is taken away and a return from the microcarrier to its original orientation is observed.

In the next experiment, the same microcarrier as in the previous experiment is used. The microcarrier is initially oriented in the magnetic field of the first magnet. After having carefully marked the position of this magnet, it is used to rotate the microcarrier by moving the magnet 360 around the reservoir and finally placing it back in its original position. The microcarrier does not return to its original orientation due to a relatively strong interaction between the polymer carrier and the glass cover slip. A second magnet is used to loosen the microcarrier by quickly moving it once near the reservoir. It is observed that the microcarrier returned immediately to its exact original orientation.

The coated particles of the present invention can be easily magnetised using a strong magnet. The microcarriers can be oriented in an external magnetic field. The orientation of the microcarriers in a certain external magnetic field is exactly reproducible after random movement of the carriers when the initial field is applied again. No difference in orientation can be observed within pixel accuracy (0.7um/pixel).

### Example 6: Use of spherical microcarriers with a single axis of symmetry for identification purposes i.e. encoding and reading in a flow cell.

The necessity of an orientation and a positioning for identification purposes is elucidated hereunder. The code on said spherical microcarrier is written along the symmetry axis, whereby the code is encoded (written) or identified (read) by means of a high spatial resolution light source, more in particular by using fluorescence bleaching.

Spherical microcarriers are oriented with their symmetry axis along the flow. The laser beam for fluorescence bleaching has a stationary position in the confocal microscope, and the code on said microcarrier is written along the symmetry axis. The flow itself serves as the scanning motion along the symmetry axis. A code written as described above (along the symmetry axis), may be read by a laser beam having a stationary position.

In the case of a stationary writing/reading laser beam, and accurate flowing of the microcarrier, the code may be written/read along the axis of symmetry of the microcarrier. However, in the case where the flow is not sufficiently reproducible with respect to the microscope focus, the code may not be read correctly wherein the code is written/read below the axis of symmetry.

An auxiliary laser beam may be used to illuminate the passing microcarrier. In this case, a shadowing effect will be observed, behind the microcarrier, due to partial absorption or reflection of light by said microcarrier. A photodiode consisting of two separated cells (bicell photodetector) is positioned at the opposite side of the flow cell in order to measure the shadowing effect. When the centre of the spherical microcarrier crosses the optical axis of the microscope, the same amount of light is collected by the 2 cells, and the bicell photodetector measures a difference signal equal to zero, indicating that the carrier passes by at the correct height. When the center of the spherical microcarrier does not cross the optical axis of the microscope, the bicell photodetector measures a difference signal different from zero, indicating that the microcarrier flows too high.

Consequently, the use of a photodiode permits the detection of a mispositioning of the microcarriers in the flow and indicates whether said microcarriers flow too high or too low from the optical axis.

This photodiode system may be used to measure the position of the microcarrier before said microcarrier arrives at the focus of the reading/writing the laser beam. In this case, the position error signal generated can be used to adjust the focus of the reading/writing beam. When the position error signal measured is zero, the position of the beam focus was not changed. An error signal is measured in this case, and the beam focus position is moved up. Adjusting the focus of the laser beam can be done by changing the direction of incidence of the writing/reading beam on the microscope objective. An acoustooptic beam deflector can be used as a device that can quickly adapt the direction of the laser beam. The same technique can be used to generate a position error signal for the Z axis, i. e. the optical axis of the microscope. Because there will be only a difference signal at the bicell photodetector, the difference signal can be used to detect the arrival of the microcarrier and can also be used as a trigger for reading and writing.

## Claims

1. A microcarrier **characterised in that** it comprises a core coated with
- at least one layer comprising a polyelectrolyte material; and
- at least one layer comprising magnetic material comprising particles of less than 500 nanometer, the magnetic material having a concentration ranging from 0.1 to 50% by weight of the microcarrier;
wherein each said at least one layer comprising magnetic material is applied on top of one of said at least one layer comprising polyelectrolyte material and wherein the microcarrier is encoded with a written code wherein said code is a spatial modulation created inside the microcarrier or on its outer surface, said spatial modulation being a known arrangement of a finite number of distinct volume elements.

2. The microcarrier according to claim 1 which comprises one single layer of magnetic particles.

3. The microcarrier according to claim 1 or 2, wherein said core comprises a bleachable material.

4. The microcarrier according to any one of claims 1 to 3, which comprises between 2 and 10 layers of polyelectrolyte material.

5. The microcarrier according to any one of claims 1 to 4, wherein the outer layer of said microcarrier is a layer comprising negatively charged polyelectrolyte material.

6. The microcarrier according to any one of claims 1 to 5, wherein said magnetic material is ferromagnetic material.

7. The microcarrier according to any one of claims 1 to 6, wherein the magnetic particles have a size between 100 and 400 nanometer.

8. The microcarrier according to any one of claims 1 to 7, wherein said microcarrier has a diameter between 10 and 100 µm.

9. The microcarrier according to any one of claims 1 to 6. wherein the magnetic particles have a size of less than 220 nanometer.

10. The microcarrier according to any one of claims 1 to 9, wherein the microcarriers are encoded in the central plane of the microcarrier.

11. The microcarrier according to any of claims 1 to 10, further comprising one or probes bound to the outer layer of a polyelectrolyte material.

12. A method for manufacturing a magnetic microcarrier, comprising the steps of
a) providing a microparticle,
b) applying a layer comprising a polyelectrolyte material,
c) applying on top of said layer of polyelectrolyte material, a layer comprising magnetic material comprising particles of less than 500 nanometer, the magnetic material having a concentration ranging from 0.1 to 50% bv weight of the microcarrier;
d) optionally repeating steps (b) and (c) one or more times, and
e) encoding the microcarrier with a code wherein the code is a spatial modulation created inside the microcarrier or on its outer surface.

13. The method of claim 12, comprising the steps of:
a) providing a microparticle,
b) applying one layer comprising a positively charged polyelectrolyte or applying a plurality of layers comprising electrolytes with alternating charges wherein the outer layer has a positive charge;
c) applying one layer of magnetic particles of less than 500 nm,
d) optionally repeating steps (b) and (c) one or more times, and
e) applying one layer comprising a positively charged polyelectrolyte or applying a plurality of layers comprising electrolytes with alternating charges comprising alternating charges wherein the inner layer has a positive charge.

14. Use a microcarrier according to any of claims 1 to 11 for analyte detection in a magnetic field.

## Patentansprüche

1. Ein Mikroträger, **dadurch gekennzeichnet, dass** dieser einen Kern umfasst, welcher mit
- mindestens einer Schicht, umfassend ein Polyelektrolytmaterial; und
- mindestens einer Schicht, umfassend ein magnetisches Material, welches Teilchen von weiniger als 500 Nanometer umfasst, wobei das magnetische Material eine Konzentration im Bereich von 0,1 bis 50 Gew.-% des Mikroträgers aufweist, beschichtet ist;
wobei jede der mindestens einen ein magnetisches Material umfassenden Schicht obenauf auf einer der mindestens einen ein Polyclektrolytmaterial umfassenden Schicht aufgebracht ist und wobei der Mikroträger mit einem geschriebenen Code kodiert ist, wobei der Code eine räumliche Modulation ist, welche innerhalb des Mikroträgers oder auf dessen äußerer Oberfläche erzeugt ist, wobei die räumliche Modulation eine bekannte Anordnung einer endlichen Zahl von unterscheidbaren Volumenelementen ist.

2. Der Mikroträger gemäß Anspruch 1, welcher eine einzelne Schicht magnetischer Teilchen umfasst.

3. Der Mikroträger gemäß Anspruch 1 oder 2, wobei der Kern ein bleichbares Material umfasst.

4. Der Mikroträger gemäß einem der Ansprüche 1 bis 3, welcher zwischen 2 und 10 Schichten Polyelektrolytmaterial umfasst.

5. Der Mikroträger gemäß einem der Ansprüche 1 bis 4, wobei die äußere Schicht des Mikroträgers eine Schicht ist, welche negativ geladenes Polyelektrolytmaterial umfasst.

6. Der Mikroträger gemäß einem der Ansprüche 1 bis 5, wobei das magnetische Material ein ferromagnetisches Material ist.

7. Der Mikroträger gemäß einem der Ansprüche 1 bis 6, wobei die magnetischen Teilchen eine Größe zwischen 100 und 400 Nanometer aufweisen.

8. Der Mikroträger gemäß einem der Ansprüche 1 bis 7, wobei der Mikroträger einen Durchmesser zwischen 10 und 100 µm aufweist.

9. Der Mikroträger gemäß einem der Ansprüche 1 bis 6, wobei die magnetischen Teilchen eine Größe von weniger als 220 Manometer aufweisen.

10. Der Mikroträger gemäß einem der Ansprüche 1 bis 9, wobei die Mikroträger in der zentralen Ebene des Mikroträger kodiert sind.

11. Der Mikroträger gemäß einem der Ansprüche 1 bis 10, ferner umfassend eine oder Proben, welche an die äußere Schicht eines Polyelektrolytmaterials gebunden sind.

12. Ein Verfahren zur Herstellung eines magnetischen Mikroträgers, umfassend die Schritte
a) Bereitstellen eines Mikroteilchens,
b) Aufbringen einer Schicht, umfassend ein Polyelektrolytmaterial,
c) Aufbringen obenauf der Polyelektrolytmaterial-Schicht eine Schicht, die ein magnetisches Material umfasst, das Teilchen von weniger als 500 Nanometer umfasst, wobei das magnetische Material eine Konzentration im Bereich von 0,1 bis 50 Gew.-% des Mikroträgers aufweist,
d) gegebenenfalls einmaliges oder mehrmaliges Wiederholen der Schritte b) und c) und
e) Kodieren des Mikroträgers mit einem Code, wobei der Code eine räumliche Modulation ist, welche innerhalb des Mikroträgers oder auf dessen äußerer Oberfläche erzeugt wird.

13. Das Verfahren gemäß Anspruch 12, umfassend die Schritte:
a) Bereitstellen eines Mikroteilchens,
b) Aufbringen einer Schicht, umfassend einen positiv geladenen Polyelektrolyt, oder Aufbringen einer Mehrzahl von Schichten, umfassend Elektrolyte mit alternierenden Ladungen, wobei die äußere Schicht eine positive Ladung aufweist,
c) Aufbringen einer Schicht magnetischer Teilchen von weniger als 500 nm,
d) gegebenenfalls einmaliges oder mehrmaliges Wiederholen der Schritte b) und c) und
e) Aufbringen einer Schicht, umfassend einen positiv geladenen Polyelektrolyt, oder Aufbringen einer Mehrzahl von Schichten, umfassend Elektrolyte mit alternierenden Ladungen, umfassend alternierende Ladungen, wobei die innere Schicht eine positive Ladung aufweist.

14. Verwendung eines Mikroträgers gemäß einem der Ansprüche 1 bis 11 zur Analytdetektion in einem magnetischen Feld.

## Revendications

1. Microsupport **caractérisé en ce qu'**il comprend un noyau revêtu avec
- au moins une couche comprenant un matériau polyélectrolyte ; et
- au moins une couche comprenant un matériau magnétique comprenant des particules de moins de 500 nanomètres, le matériau magnétique ayant une concentration allant de 0,1 à 50 % en poids du microsupport ;
dans lequel chacune de ladite au moins une couche comprenant un matériau magnétique est appliqué sur le dessus de l'une de ladite au moins une couche comprenant un matériau polyélectrolyte et dans lequel le microsupport est codé avec un code écrit dans lequel ledit code est une modulation spatiale créée à l'intérieur du microsupport ou sur sa surface externe, ladite modulation spatiale étant un agencement connu d'un nombre fini d'éléments de volumes distincts.

2. Microsupport selon la revendication 1, qui comprend une seule couche de particules magnétiques.

3. Microsupport selon la revendication 1 ou 2, dans lequel ledit noyau comprend un matériau blanchissable.

4. Microsupport selon l'une quelconque des revendications 1 à 3, qui comprend entre 2 et 10 couches de matériau polyélectrolyte.

5. Microsupport selon l'une quelconque des revendications 1 à 4, dans lequel la couche externe dudit microsupport est une couche comprenant un matériau polyélectrolyte chargé négativement.

6. Microsupport selon l'une quelconque des revendications 1 à 5, dans lequel ledit matériau magnétique est un matériau ferromagnétique.

7. Microsupport selon l'une quelconque des revendications 1 à 6, dans lequel les particules magnétiques ont une taille comprise entre 100 et 400 nanomètres.

8. Microsupport selon l'une quelconque des revendications 1 à 7, dans lequel ledit microsupport a un diamètre compris entre 10 et 100 µm.

9. Microsupport selon l'une quelconque des revendications 1 à 6, dans lequel les particules magnétiques ont une taille de moins de à 220 nanomètres.

10. Microsupport selon l'une quelconque des revendications 1 à 9, dans lequel les microsupports sont codés dans le plan médian du microsupport.

11. Microsupport selon l'une quelconque des revendications 1 à 10, comprenant en outre une ou plusieurs sondes liées à la couche externe d'un matériau polyélectrolyte.

12. Procédé de fabrication d'un microsupport magnétique, comprenant les étapes de
a) fourniture d'une microparticule,
b) application d'une couche comprenant un matériau polyélectrolyte,
c) application sur le dessus de ladite couche de matériau polyélectrolyte, d'une couche comprenant un matériau magnétique comprenant des particules de moins de 500 nanomètres, le matériau magnétique ayant une concentration allant de 0,1 à 50 % en poids du microsupport ;
d) répétition, éventuellement, des étapes (b) et (c) une ou plusieurs fois, et
e) codage du microsupport avec un code dans lequel le code est une modulation spatiale créée à l'intérieur du microsupport ou sur sa surface externe.

13. Procédé selon la revendication 12, comprenant les étapes de :
a) fourniture d'une microparticule,
b) application d'une couche comprenant un polyélectrolyte chargé positivement ou application d'une pluralité de couches comprenant des électrolytes avec des charges alternées, dans laquelle la couche externe a une charge positive ;
c) application d'une couche de particules magnétiques de moins de 500 nm,
d) répétition, éventuellement, des étapes (b) et (c) une ou plusieurs fois, et
e) application d'une couche comprenant un polyélectrolyte chargé positivement ou application d'une pluralité de couches comprenant des électrolytes avec des charges alternées comportant des charges alternées dans laquelle la couche interne a une charge positive.

14. Utilisation d'un microsupport selon l'une quelconque des revendications 1 à 11 pour la détection d'un analyte dans un champ magnétique.
